# EUROPEAN PATENT APPLICATION

(11) **EP 4 130 262 A1**
(43) Date of publication of application: **08.02.2023**
(21) Application number: 22790431.5
(22) Date of filing: 31.05.2022
(51) Int. Cl.: C12N 15/10

(54) **PLANT CELLS HAVING ENGINEERED QPT GENE AND METHOD FOR USING SAME**

(30) Priority: 21.06.2021 KR 20210080363
(71) Applicant: KT & G Coporation, Daejeon 34337 (KR)
(72) Inventor: SEO, Hyo Seok, Daejeon 34128 (KR); LEE, Young Gi, Daejeon 34128 (KR); LEE, Jeong Heon, Daejeon 34128 (KR); JANG, Dong Sung, Daejeon 34128 (KR); KIM, Kwang Chul, Daejeon 34128 (KR); NA, Woong Hyun, Daejeon 34128 (KR)
(74) Representative: Ter Meer Steinmeister & Partner
(86) International application number: PCT/KR2022/007757
(87) International publication number: WO 2022/270788

(57) **Abstract**

An aspect provides plant cells with an engineered QPT gene and methods of using the same. When a QPT gene in a plant cell is genetically engineered according to an aspect, biosynthesis of nicotine is effectively inhibited, and nicotine is reduced by about 97 % or more, and since alkaloids other than nicotine (nornicotine, anabasine, anatabine) are also reduced by about 68 % or more, plant cells with reduced carcinogen TSNAs (NNK, NNN, NAB, and NAT) may be produced.

## Description

### TECHNICAL FIELD

The present disclosure relates to plant cells with an engineered QPT gene and methods of using the same.

### BACKGROUND ART

A quinolinic acid phosphoribosyl transferase (QPT) gene is known to play an essential role in nicotine biosynthesis in tobacco plants, a QPT1 gene has been identified as an essential gene for plant growth, and a QPT2 gene was found to have selectively increased expression during nicotine biosynthesis (Ryan et al., 2012).

On the other hand, in the related art, nicotine content was lowered by suppressing the function of a gene related to nicotine biosynthesis in tobacco plants through GMO technology (RNAi, etc.). However, due to limitations of the previous technology, it was not possible to suppress the gene function 100 %, and there was a limit to lowering the nicotine content.

This led to the development of a plant which is a "non-GMO with no external gene introduced" and "tobacco with a remarkably low nicotine content" by inducing a mutation in the QPT2 gene, a gene involved in nicotine biosynthesis in tobacco plants, by using a CRISPR/Cas system.

### DESCRIPTION OF EMBODIMENTS

### TECHNICAL PROBLEM

An aspect provides plant cells genetically engineered to have reduced expression or activity of a quinolinic acid phosphoribosyl transferase (QPT) gene or a QPT protein.

An aspect provides a plant including the plant cell.

An aspect provides a CRISPR/Cas system including: at least one first polynucleotide selected from the group consisting of polynucleotides including a polynucleotide including nucleotide sequences of SEQ ID NOS: 9 to 19; or a second polynucleotide into which the at least one first polynucleotide is transcribed.

Another aspect provides a composition for inhibiting alkaloid biosynthesis, a composition for inhibiting nicotine biosynthesis, and a composition for engineering the QPT gene.

Another aspect provides a method of producing plant cells in which nicotine biosynthesis is inhibited and a method of engineering QPT genes of plant cells.

### SOLUTION TO PROBLEM

An aspect provides plant cells genetically engineered to have reduced expression or activity of a quinolinic acid phosphoribosyl transferase (QPT) gene or a QPT protein.

The term "parent cell" refers to a cell that has not been artificially manipulated to reduce the expression or activity of the QPT gene or QPT protein according to an aspect, and refers to a cell freshly isolated from a plant and a cell culture thereof.

The QPT gene may be a gene related to nicotine biosynthesis, and the QPT gene may be a QPT gene derived from *Nicotiana sylvestris,* a QPT gene derived from *Nicotiana tomentosiformis* and/or a QPT gene of *Nicotiana tabacum.* For example, the QPT gene may be at least one gene selected from the group consisting of QPT1s, QPT1t, QPT2s and QPT2t. The QPT1s gene may be a QPT1 gene derived from N. *sylvestris,* the QPT2s gene may be a QPT2 gene derived from *N. sylvestris,* the QPT1t gene may be a QPT1 gene derived from *N. tomentosiformis*, and the QPT2t gene may be a QPT2 gene derived from *N. tomentosiformis.* The QPT2s may be used interchangeably with NtQPT2s, NtQPT2syl or QPT2_syl in the present specification, and the QPT2t may be used interchangeably with NtQPT2t, NtQPT2tom, or QPT2_tom in the present specification.

The QPT genes may respectively correspond to sequence IDs of AJ748263, AJ748262, NW_009541109.1, NW_015842103.1, NW_008919084.1, NW_015852968.1, NW_009350226, NW_015824186.1, NW_008919267.1, NW_015852968.1, NW_015842103.1, NW_015824186.1, NW_015852968.1, and NW_015852968.1, which are currently registered in NCBI GenBank. In addition, the QPT gene may respectively correspond to gene IDs of 107825849, 104240014, 107829123, 107829122, 104217269, 107820078, 104121046, 104121140, which are currently registered in NCBI GenBank. A person skilled in the art will be able to easily identify the sequences using the sequence IDs or gene ID accession numbers. Specific sequences corresponding to Sequence ID numbers registered in the UCSC genome browser or GenBank may be slightly changed over time. It will be apparent to those skilled in the art that the scope of the present disclosure also extends to such altered sequences. More specifically, the QPT gene is a QPT2 gene expressed in response to a wound or methyl jasmonate treatment, which are signals for inducing nicotine biosynthesis, and may include at least one gene selected from the group consisting of QPT2s and QPT2t.

In addition, the term "genetic engineering" or "genetically engineered", used herein, refers to an act of introducing at least one genetic modification into a cell or a cell thereby made.

The genetic engineering may be induced by modification in the nucleotide sequence of the QPT gene by a physical method. The physical method may be, for example, X-ray irradiation, gamma-ray irradiation, or the like.

The genetic engineering may be induced by a chemical method by a change in the nucleotide sequence of the QPT gene or a change in the expression of the gene. The chemical method may be, for example, ethyl methanesulfonate treatment, dimethyl sulfate treatment, or the like.

The genetic engineering may be induced by modification in the nucleotide sequence of the QPT gene by a gene editing system. The gene editing system may be, for example, a meganuclease system, a zinc finger nuclease system, a transcription activator-like effector nuclease (TALEN) system, a CRISPR/Cas system, and the like. For example, the genetic engineering may be induced by a change in gene expression by binding to mRNA transcribed from the QPT gene by an RNA interference (RNAi) system.

Accordingly, the genetic engineering performed in the plant cell may be induced by at least one selected from the group consisting of an RNA interference (RNAi) system, a meganuclease system, a zinc finger nuclease system, and a TALEN system, a CRISPR/Cas system, X-ray irradiation, gamma-ray irradiation, ethyl methanesulfonate treatment, and dimethyl sulfate treatment.

The term "reduced expression or activity" of a QPT gene or a protein encoded by the QPT gene or "inactivation" of the QPT gene, "reduced expression or activity" of the QPT protein or genetically engineered plant cells where the QPT gene is "inactivated" means the QPT gene or the protein encoded by the QPT gene has no expression or activity or exhibits expression or activity to a lower degree than the expression or activity level of the QPT gene or the protein encoded by the QPT gene measured in comparable plant cells of the same species or the parent cells. That is, in plant cells, the expression or activity of the QPT gene or the protein encoded by the QPT gene may be reduced by about 20 % or more, about 30 % or more, about 40 % or more, about 50 % or more, about 55 % or more, about 60 % or more, about 70 % or more, about 75 % or more, about 80 % or more, about 85 % or more, about 90 % or more, about 95 % or more, or about 100 %, compared to the expression or activity of unengineered plant cells. Genetically engineered plant cells with reduced expression or activity of the QPT gene or the protein encoded by the QPT gene may be confirmed by using any method known in the art. The term "inactivation" may mean generation of a gene that is not expressed at all or a protein that has no activity even when the protein is expressed. The term "depression" may mean that the QPT gene is expressed at a lower level compared to unengineered plant cells, or that the activity of the protein encoded by the QPT gene is low even when the protein is expressed.

The genetic engineering may be induced by modification in the nucleic acid sequence of the QPT gene, and the modification in the nucleic acid sequence may occur artificially by the CRISPR/Cas system.

The second polynucleotide included in the CRISPR/Cas system may be a single guide RNA (sgRNA) including a CRISPR RNA (crRNA) and a transactivating crRNA (tracrRNA), and the first polynucleotide may be a gene that encodes the second polynucleotide.

In general, "clustered regularly interspaced short palindromic repeats (CRISPR) system", which is a widely known means of gene editing, may collectively refer to a sequence encoding a Cas gene, a trans-activating CRISPR (tracr) sequence, (for example, tracrRNA or active moiety tracrRNA), a tracr-mate sequence (includes "direct repeat" in the context of an endogenous CRISPR system, and direct repeat in tracrRNA-processing moiety), a guide sequence (also referred to as "spacers" in the context of an endogenous CRISPR system), and transcripts and other elements involved in the expression of a CRISPR-associated (hereinafter Cas) gene, or inducing activity of the same, including guide RNA or other sequences and transcrips from the CRISPR gene loci. In some embodiments, one or more elements of the CRISPR system are derived from a type I, type II, or type III CRISPR system. In some embodiments, at least one element of the CRISPR system is derived from a particular organism including an endogenous CRISPR system, for example, *Streptococcus pyogenes.* In general, CRISPR systems are characterized by elements (also referred to as protospacers in the context of endogenous CRISPR systems) that promote the formation of CRISPR complexes at the site of the target sequence. In the context of formation of a CRISPR complex, a "target sequence" or "target gene" refers to a sequence designed to have complementarity with a guide sequence, wherein hybridization between the target sequence and the guide sequence enhances formation of a CRISPR complex. Although essentially perfect complementarity is not required, there is sufficient complementarity to cause hybridization and promote formation of a CRISPR complex. A target sequence may include any polynucleotide, for example, DNA or RNA polynucleotide. In some embodiments, the target sequence is located in the nucleus or cytoplasm of the cell. In some embodiments, the target sequence may be present in an organelle of a eukaryotic cell, for example, inside a mitochondrion or a chloroplast.

The CRISPR/Cas system may include a Cas protein and or a variant thereof. When the Cas protein forms a complex with two RNAs called CRISPR RNA (crRNA) and trans-activating crRNA (tracrRNA), the protein forms an active endonuclease or nickase. Non-limiting examples of the Cas protein include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, or a homologue thereof or a modified version thereof. These enzymes are known; for example, the amino acid sequence of *Streptococcus pyogenes* Cas9 protein may be obtained from the SwissProt database under an accession number of Q99ZW2. In some embodiments, an unmodified CRISPR enzyme, for example, Cas9, has DNA cleavage activity.

In addition, the CRISPR/Cas system may include a Cas protein selected from the group consisting of Cas1, Cas1B, Cas2, Cas3, Cas4, Cas5, Cas6, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and Cpf1; or a gene encoding the protein, and a nuclear localization signal (NLS) protein or a gene encoding the NLS protein.

Specifically, the CRISPR/Cas system may include the first polynucleotide, a CRISPR associated protein 9 (Cas9) protein or a gene encoding the Cas9 protein, and a nuclear localization signal (NLS) protein or a gene encoding the NLS protein.

In some embodiments, the CRISPR enzyme may use a CRISPR/Cas9 system, which is a Cas9 protein. The Cas9 protein may be at least one Cas9 protein selected from the group consisting of a Cas9 protein derived from *Streptococcus pyogenes,* a Cas9 protein derived from *Campylobacter jejuni,* a Cas9 protein derived from *Streptococcus thermophiles,* a Cas9 protein derived from *Streptocuccus aureus,* and a Cas9 protein derived from *Neisseria meningitidis,* or specifically, may be a Cas9 protein derived from *Streptococcus pyogenes.* In some embodiments, the Cas9 protein is codon-optimized for expression in a eukaryotic cell, and when the Cas9 protein derived from *Streptococcus pyogenes* is used, expression or activity of the QPT gene or QPT protein may be maximally reduced.

In some embodiments, a Cas9 protein may include a nuclear localization sequence or signal (NLS) at the 5'-end or 3'-end, or both ends of the Cas9 protein for localization in the nucleus in a eukaryotic cell, wherein the NLS may be one or more.

The term "nuclear localization sequence or signal (NLS)", used herein, refers to an amino acid sequence that serves to transport a specific substance (for example, a protein) into the cell nucleus, and generally acts to deliver the substance into the cell nucleus through a nuclear pore. The nuclear localization sequence is not required for CRISPR complex activity in eukaryotes, however, it is believed that the inclusion of such sequences enhances the activity of the system, and particularly targets nucleic acid molecules in the nucleus.

In addition, an RNA-guided clustered regularly interspaced short palindrome repeats (CRISPR)-associated nuclease Cas9 provides a groundbreaking technology for knockout of target genes, transcriptional activation and inhibition using single guide RNAs (sgRNAs) (that is, crRNA-tracrRNA fusion transcript), and the technology is known to target numerous gene loci.

Cas9 (or Cpf1) protein refers to an essential protein element in a CRISPR/Cas system, and information on the Cas9 (or Cpf1) gene and protein may be obtained from GenBank of national center for biotechnology information, (NCBI), but is not limited thereto. CRISPR-associated genes encoding a Cas (or Cpf1) protein are known to have about 40 or more different Cas (or Cpf1) protein families, and 8 CRISPR subtypes (Ecoli, Ypest, Nmeni, Dvulg, Tneap, Hmari, Apern, and Mtube) may be defined according to specific combinations of Cas genes and repeat structures. Therefore, each of the CRISPR subtypes may form a repeating unit to form a polyribonucleotide-protein complex.

The gene knockout may refer to regulation of gene activity, for example, inactivation, by deletion and substitution of all or part of a gene (for example, one or more nucleotides), and/or insertion of one or more nucleotides. The gene inactivation refers to a modification to encode a protein that has suppressed or downregulated expression of a gene or has lost its original function. Also, gene regulation may refer to a change in gene function as a result of structural modification of the protein obtained by deletion of the exon site due to simultaneous targeting of both intron sites surrounding one or more exons of the target gene, expression of a protein in a dominant negative form, expression of a competitive inhibitor secreted in a soluble form, etc. Specifically, the second polynucleotide may bind to at least one site in the region consisting of Exons 1 to 8 of the QPT gene, and more specifically, the second polynucleotide may bind to sites of Exons 1 to 8 of the QPT2 gene, and even more specifically, the second polynucleotide may bind to sites of Exons 2 and 3 of the QPT2 gene.

Genetic engineering artificially performed to reduce expression or activity of the QPT gene or QPT protein may be induced by a Cas9 protein or a Cpf1 protein. Plant cells according to an aspect may be genetically engineered to reduce expression or activity of a quinolinic acid phosphoribosyl transferase (QPT) gene or a QPT protein compared to the parent cell, which may be accomplished by a CRISPR/Cas system, and the CRISPR/Cas system may include a CRISPR/Cas system including at least one first polynucleotide selected from the group consisting of polynucleotides including nucleotide sequences of SEQ ID NOS: 9 to 19; or a second polynucleotide into which the at least one first polynucleotide is transcribed.

Accordingly, in plant cells according to an aspect, the CRISPR/Cas system may be delivered to the plant cells to inactivate the QPT gene involved in nicotine biosynthesis, and modified QPT proteins are expressed, thereby inhibiting nicotine biosynthesis.

The reduction in the expression or activity of the QPT gene or QPT protein may be due to a mutation, substitution, or deletion of a part of or all of the gene encoding the QPT, or an insertion of at least one base to the gene; and may be by a CRISPR/Cas system, a means of QPT gene editing.

When the knocked-out QPT genes are QPT2s and QPT2t, or the two genes are all knocked-out, nicotine biosynthesis may be maximally inhibited.

The plant may be *Nicotiana tabacum,* or specifically, a flue-cured species, a burley species, a native species, a black tobacco species or an orient species, and more specifically, a burley species or a flue-cured species.

When the Cas protein is encoded by DNA and delivered to a subject or cell, the DNA may generally (but not necessarily) include a regulatory element (for example, a promoter) operable in the target cell. A promoter for expression of the Cas protein may be, for example, a CMV, EF-1 a, EFS, MSCV, PGK, or a CAG promoter. A promoter for gRNA expression may be, for example, a HI, EF-la, tRNA or U6 promoter. In addition, the sequence of the gene encoding Cas9 among the Cas proteins may include a nuclear localization signal (NLS) (for example, SV40 NLS). In an example, the promoter may have tissue specificity or cell specificity.

Genetic engineering artificially performed to reduce expression or activity of a QPT gene or QPT protein may be by a deletion of all of the gene within the protospacer-adjacent motif (PAM) sequence in the nucleic acid sequence constituting the QPT gene, or within a continuous 1 bp to 50 bp nucleotide sequence region located adjacent to the 5' end or 3' end of the PAM, or a deletion of at least one nucleotide in the region of continuous nucleotide sequence; a substitution with a nucleotide different from the wild-type gene; insertion of 1 to 23 nucleotides each independently selected from A, T, C and G; or a combination of the above modifications.

In an embodiment, the second polynucleotide may be one bound to the QPT gene of at least one allele in the plant cell, or specifically, the second polynucleotide may be one that binds to the QPT gene of all alleles. When the second polynucleotide binds to the QPT gene of all alleles to knock out the QPT gene, nicotine biosynthesis may be maximally inhibited not only in the same generation but also in the plant cells of subsequent generations.

In an embodiment, a target sequence used to knock out the QPT gene in the plant cell may be, for example, at least one region in the region consisting of Exons 1 to 8 of the QPT gene, or more specifically, a region of Exons 1 to 8 of the QPT 2 gene.

The genetic engineering artificially performed to reduce expression or activity of the QPT gene or QPT protein may be to prevent the protein translated from the QPT gene from being expressed in a form of a protein having an original function. The genetic engineering may be induced by one or more of the following:
1) a deletion of all or part of the QPT gene, for example, 1bp or more nucleotides of the QPT gene, for example, deletion of 1 to 30, 1 to 27, 1 to 25, 1 to 23, 1 to 20, 1 to 15, 1 to 10, 1 to 5, 1 to 3, or 1 nucleotide.
2) substitution of 1 bp or more nucleotides of the QPT gene, for example, 1 to 30, 1 to 27, 1 to 25, 1 to 23, 1 to 20, 1 to 15, 1 to 10, 1 to 5, 1 to 3, or 1 nucleotide with nucleotides different from the original (wild-type);
3) insertion of at least one nucleotide (each independently selected from A, T, C and G) at any position in the target gene, for example, insertion of 1 to 30, 1 to 27, 1 to 25, 1 to 23, 1 to 20, 1 to 15, 1 to 10, 1 to 5, 1 to 3, or 1 nucleotide; and
4) a combination of two or more selected from 1) to 3) above.

The modified part of the QPT gene ('target site') may be a part of the continuous nucleotide sequence of 1 bp or more, 3 bp or more, 5 bp or more, 7 bp or more, 10 bp or more, 12 bp or more, 15 bp or more, 17 bp or more, 20 bp or more, for example, 1 bp to 30 bp, 3 bp to 30 bp, 5 bp to 30 bp, 7 bp to 30 bp, 10 bp to 30 bp, 12 bp to 30 bp, 15 bp to 30 bp, 17 bp to 30 bp, 20 bp to 30 bp, 1 bp to 27 bp, 3 bp to 27 bp, 5 bp to 27 bp, 7 bp to 27 bp, 10 bp to 27 bp, 12 bp to 27 bp, 15 bp to 27 bp, 17 bp to 27 bp, 20 bp to 27 bp, 1 bp to 25 bp, 3 bp to 25 bp, 5 bp to 25 bp, 7 bp to 25 bp, 10 bp to 25 bp, 12 bp to 25 bp, 15 bp to 25 bp, 17 bp to 25 bp, 20 bp to 25 bp, 1 bp to 23 bp, 3 bp to 23 bp, 5 bp to 23 bp, 7 bp to 23 bp, 10 bp to 23 bp, 12 bp to 23 bp, 15 bp to 23 bp, 17 bp to 23 bp, 20 bp to 23 bp, 1 bp to 20 bp, 3 bp to 20 bp, 5 bp to 20 bp, 7 bp to 20 bp, 10 bp to 20 bp, 12 bp to 20 bp, 15 bp to 20 bp, 17 bp to 20 bp, 21 bp to 25 bp, 18 bp to 22 bp, or 21 bp to 23 bp.

In an example, the gene knockout may be to reduce expression of the QPT gene, which is the target gene, by catalyzing single-stranded or double-stranded cleavage at a specific site within the target gene by using a genome editing system including a rare-cutting endonuclease. Nucleic acid strand breaks catalyzed by the rare-cutting endonuclease may be repaired through mechanisms such as homologous recombination or non-homologous end joining (NHEJ). In this case, when NHEJ mechanism occurs, a change is induced in the DNA sequence at the cleavage site, and thus, the gene may be inactivated. Repair through NHEJ results in substitutions, insertions or deletions of short gene fragments, and may be used to induce gene knockouts. The modification may be substitutions, deletions, and/or insertions of one or more nucleotides, for example, nucleotides of 1 bp to 30 bp, 1 bp to 27 bp, 1 bp to 25 bp, 1 bp to 23 bp, 1 bp to 20 bp, 1 bp to 15 bp, 1 bp to 10 bp, 1 bp to 5 bp, 1 bp to 3 bp, or 1 bp.

The rare-cutting endonuclease may be at least one selected from the group consisting of a meganuclease, a zinc finger nuclease, CRISPR/Cas9 (a Cas9 protein), CRISPR-Cpf1 (a Cpf1 protein) and a TALE-nuclease. In an embodiment, the rare cleavage endonuclease may be a Cas9 protein or a Cpf1 protein.

The terms "chimeric RNA", "chimeric guide RNA", "guide RNA", "single guide RNA (sgRNA)" and "synthetic guide RNA" are used interchangeably, and refer to a polynucleotide sequence including a guide sequence, a tracr sequence and/or a tracr mate sequence. The term "guide sequence" refers to about a 20 bp sequence within a guide RNA that designates a target site, and may be used interchangeably with the terms "guide" or "spacer". In addition, the term "tracr mate sequence" may be used interchangeably with the term "direct repeat(s)". The guide RNA may be composed of two RNAs, that is, CRISPR RNA (crRNA) and transactivating crRNA (trRNA), or may be a single-chain RNA (sgRNA) including a portion of crRNA and tracrRNA, and hybridizing with the target DNA.

In general, a guide sequence is any polynucleotide sequence that has enough complementarity with the target polynucleotide sequence to hybridize with the target sequence and to induce sequence-specific binding of a CRISPR complex to a target sequence. In addition, any nucleotide sequence that may be used in genetic engineering to reduce the expression or activity of the QPT gene or QPT protein may be used as a guide RNA without limitation, and for example, the nucleotide sequence may be a sequence capable of hybridizing with the QPT gene. In addition, a portion of the guide RNA nucleotide sequence may be modified in order to modify/enhance the function of the guide RNA. In addition, in some embodiments, the degree of complementarity between a guide sequence and its corresponding target sequence, when optimally aligned using an appropriate alignment algorithm, may be about 50 % or more, 60 % or more, 75 % or more, 80 % or more, 85 % or more, 90 % or more, 95 % or more, 97.5 % or more, or 99 % or more. An optimal alignment may be determined by using any algorithm suitable for aligning sequences, and non-limiting examples thereof include the Smith-Waterman algorithm, the Needleman-Wunsch algorithm, algorithms based on the Burrows-Wheeler Transform (for example, Burrows Wheeler Alilgner), ClustalW, Clustal X, BLAT, Novoalign (Novocraft Technologies), ELAND (Illumina, San Diego, CA, USA), SOAP (available at soap.genomics. org.cn) and Maq (available at maq.sourceforge.net). In some embodiments, the guide sequence may be, about 5 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 35 or more, 40 or more, 45 or more, 50 or more, or 75 or more nucleotides in length. In some embodiments, the guide sequence may be no more than about 75, 50, 45, 40, 35, 30, 25, 20, 15, 12 nucleotides in length. The ability of a guide sequence to induce sequence-specific binding of a CRISPR complex to a target sequence may be assessed by any suitable assay. For example, components of a CRISPR system sufficient to form a CRISPR complex including the guide sequence being tested may be provided as, for example, a host cell having the corresponding target sequence, after transfection with a vector encoding the component of the CRISPR sequence, and for example, as by assessment of preferential cleavage in the target sequence by a surveyor assay as described herein. Similarly, cleavage of a target polynucleotide sequence may be assessed *in vitro,* by providing components of a CRISPR complex including a target sequence, a guide sequence to be tested, and a control group guide sequence different from the test guide sequence, and comparing the rates of binding or cleavage between reactions at the target sequence of the guide sequences of the test group and the control group. Other assays are possible and will be readily available to those skilled in the art.

A guide sequence may be selected to target any target sequence. In some embodiments, a target sequence is a sequence within the genome of a cell. Exemplary target sequences include those that are unique in the target genome. A guide sequence of an aspect may be at least one selected from the group consisting of polynucleotides including a polynucleotide consisting of nucleotide sequences of SEQ ID NOS: 9 to 19, specifically, may correspond to sg5 including the polynucleotide consisting of SEQ ID NO: 13 and/or sg7 including the polynucleotide consisting of SEQ ID NO: 15. The CRISPR/Cas9 system may include a polynucleotide including at least one nucleotide sequence of SEQ ID NOS: 9 to 19, specifically, may include a polynucleotide including one, two, or three or more nucleotide sequences of SEQ ID NOS: 9 to 19.

In an embodiment, the genetic engineering may be performed by transforming with a vector including the first polynucleotide, the gene encoding the Cas9 protein, and the gene encoding the NLS.

For the transformation, the method is not particularly limited as long as the method is a transformation method well known in the art that allows transformation of a gene of a *Nicotiana tabacum,* but as a specific example, the transformation may be carried out by one selected from the group consisting of a agrobacterium-mediated transformation method, a polyethylene glycol (PEG)-mediated protoplast transformation method, a gene gun method, an electrode transformation method, a vacuum infiltration transformation method and a silicon carbide fiber-mediated transformation method, and considering the characteristics and transformation rate of rice, agrobacterium-mediated transformation may be used.

The term "vector" refers to a means for expressing a gene of interest in a host cell. For example, viral vectors such as plasmid vectors, cosmid vectors and bacteriophage vectors, adenoviral vectors, retroviral vectors and adeno-associated viral vectors are included. A vector that may be used as the recombinant vector may be constructed by engineering a plasmid (for example, V2k_GE, pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), a phage or a virus (for example, SV40, etc.) often used in the art.

In the vector, the first polynucleotide, the gene encoding the Cas protein or a variant thereof, and the gene encoding the NLS may be operably linked to a promoter. The term "operatively linked" refers to a functional linkage between a nucleotide expression regulatory sequence (for example, a promoter sequence) and another nucleotide sequence. Such regulatory sequences may be "operatively linked" to regulate the transcription and/or translation of other nucleotide sequences.

The vector may be constructed, typically as a vector for cloning or as a vector for expression. The expression vector may be a vector in the art used to express an extragenous protein in plants, animals or microorganisms in the art. The vector may be constructed through various methods known in the art.

The vector may be constructed using a prokaryotic cell or a eukaryotic cell as a host. For example, when the vector used is an expression vector and a prokaryotic cell is used as a host, it is common to include a powerful promoter capable of proceeding transcription (for example, a CMV promoter, a trp promoter, a lac promoter, a tac promoter, a T7 promoter, etc.), a ribosome binding site for initiation of translation and a transcription/translation termination sequence. When eukaryotic cells are used as hosts, an origin of replication that operates in eukaryotic cells included in the vector may include, an f1 origin of replication, an SV40 origin of replication, a pMB1 origin of replication, an adeno origin of replication, an AAV origin of replication, and a BBV origin of replication, but is not limited thereto. In addition, promoters derived from genomes of mammalian cells (for example, a metallothionein promoter) or promoters derived from mammalian viruses (for example, an adenovirus late promoter, a vaccinia virus 7.5K promoter, a SV40 promoter, a cytomegalovirus promoter and a tk promoter of HSV) may be used, and the promoter generally has a polyadenylation sequence as a transcription termination sequence.

Another aspect provides a plant including the plant cell.

The plant may be a plant in which biosynthesis of alkaloids including nicotine, or nornicotine, anabasine and anatabine is inhibited in the same generation as well as in subsequent generations, thereby, it is possible to obtain a plant in which the production of alkaloids including nicotine or nornicotine, anabasine and anatabine is reduced continuously.

In an embodiment, the plant may be a plant in which QPT2s and QPT2t are simultaneously mutated.

An aspect provides a CRISPR/Cas system including: at least one first polynucleotide selected from the group consisting of polynucleotides including nucleotide sequences of SEQ ID NOS: 9 to 19; or a second polynucleotide into which the at least one first polynucleotide is transcribed.

The CRISPR/Cas9 system may include a Cas (CRISPR-associated) protein or a gene encoding a Cas protein; and a nuclear localization signal (NLS) protein or a gene encoding the NLS protein.

The Cas protein included in the CRISPR/Cas9 system may include, for example, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, a homologue thereof, or a variant thereof.

In addition, the CRISPR/Cas system is one that genetically engineers to reduce expression or activity of a QPT gene or a QPT protein in plant cells, and the QPT gene may be a QPT gene (NtQPTs) derived from *Nicotiana sylvestris,* and a QPT gene (NtQPTt) derived from *Nicotiana tomentosiformis* or a combination of NtQPTs and NtQPTt. Specifically, the NtQPTs may be QPT2s, and the NtQPTt may be QPT2t.

Another aspect provides a composition including the CRISPR/Cas system for inhibiting biosynthesis of at least one alkaloid selected from the group consisting of nornicotine, anatabine, and anabasine and for inhibiting biosynthesis of nicotine.

The composition includes a CRISPR/Cas system including at least one polynucleotide selected from the group consisting of polynucleotides including nucleotide sequences of SEQ ID NOS: 9 to 19 as a guide RNA, and may induce homozygous mutations in QPT2s and QPT2t genes, and thus, biosynthesis of nicotine may be effectively inhibited, and alkaloids including nornicotine, anabasine, and anatabine other than nicotine may also be reduced by about 68 % or more. Accordingly, the composition is capable of inhibiting even TSNAs (NNK, NNN, NAB, NAT), which are carcinogens.

Another aspect provides a composition for genetically engineering QPT including the CRISPR/Cas system.

The engineering may induce homozygous mutations in both QPT2s and QPT2t genes.

The Cas protein included in the CRISPR/Cas9 system may include, for example, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, a homologue thereof, or a variant thereof.

Another aspect provides uses of inhibiting biosynthesis including the CRISPR/Cas system of at least one alkaloid selected from the group consisting of nornicotine, anatabine, and anabasine, inhibiting biosynthesis of nicotine, and/or genetically engineering QPT.

An aspect provides a method of producing plant cells in which nicotine biosynthesis is inhibited including introducing into a plant cell a vector including a CRISPR/Cas system including: at least one first polynucleotide selected from the group consisting of polynucleotides including nucleotide sequences of SEQ ID NOS: 9 to 19; or a second polynucleotide into which the at least one first polynucleotide is transcribed.

An aspect provides a method of genetically engineering QPT of plant cells including introducing into a plant cell a vector including a CRISPR/Cas system including: at least one first polynucleotide selected from the group consisting of polynucleotides including nucleotide sequences of SEQ ID NOS: 9 to 19; or a second polynucleotide into which the at least one first polynucleotide is transcribed.

The plant cells produced by the above method in which nicotine biosynthesis is inhibited may be *Nicotiana tabacum.*

In addition, when a QPT genetic engineering method through the above-mentioned method is utilized, NtQPTs and/or NtQPTt genes in *Nicotiana tabacum* plant cells may be effectively inhibited and expression or activity of a QPT protein may be reduced, and thus, it is possible to inhibit biosynthesis of at least one alkaloid selected from the group consisting of nicotine or nornicotine, anatabine and anabasine.

Redundant content is omitted in consideration of the complexity of the present specification, and terms not defined otherwise in the present specification have the meanings commonly used in the art to which the present disclosure pertains.

### ADVANTAGEOUS EFFECTS OF DISCLOSURE

When a QPT gene in a plant cell is genetically engineered according to an aspect, biosynthesis of nicotine is effectively inhibited, and nicotine is reduced by about 97 % or more, and since alkaloids other than nicotine (nornicotine, anabasine, anatabine) are also reduced by about 68 % or more, plant cells with reduced carcinogen TSNAs (NNK, NNN, NAB, and NAT) may be produced.

### BRIEF DESCRIPTION OF DRAWINGS

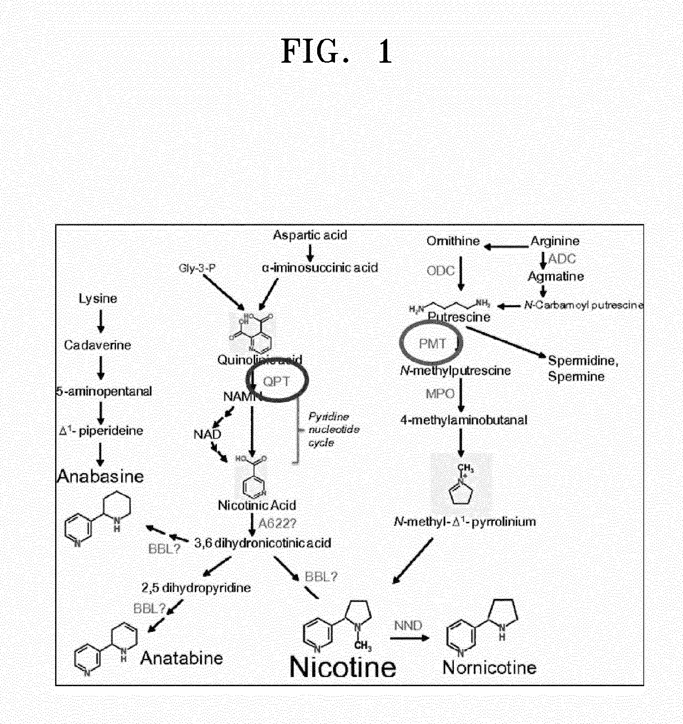

FIG. 1 is a diagram showing the nicotine biosynthesis pathway occurring in tobacco plants.
FIG. 2 is a diagram showing the results of comparing QPT genes published in the NCBI gene bank.
FIG. 3 is a diagram showing a plant-expressed gene carrier (V2k_GE) used to construct a gene carrier.
FIG. 4 is diagrams showing a plant-expressed final gene carrier and an expression block of plant expression, specifically, FIG. 4A shows V2k_GE_Q2, an expression vector for a gene carrier plant, and FIG. 4B is a diagram showing a scheme of geneic scissors and a genetic scissors guide included in a final gene carrier, which is a genetic scissors-expression block.
FIG. 5 is a diagram showing pictures of a plant tissue culture at each stage, specifically, FIG. 5A shows the stage of cutting a leaf tissue and co-culturing the same with agrobacterium for transformation, FIG. 5B shows the stage of inducing callus differentiation and shoot differentiation, FIG. 5C shows the stage of inducing root differentiation, and FIG. 5D shows the state of a plantlet in which differentiation is completed.
FIG. 6 is diagrams showing the QPT2_syl gene sequencing profile of the wild type (FIG. 6A) and the mutant No. 9 (FIG. 6B) (a portion where a single base is inserted is indicated by a red rectangle).
FIG. 7 is a diagram showing results of real-time PCR for confirming the presence of a transgene in the F1 generation mutant plant.
FIG. 8 is a diagram showing a mutation pattern of a QPTs gene of a burley species control group (WT), and a mutant (MT), specifically, it is a diagram confirming that a stop codon (asterisk) is formed abnormally early by converting the DNA nucleotide sequence (top) into an amino acid sequence (bottom).
FIG. 9 is a diagram showing a mutation pattern of a QPTt gene of a burley species control group (WT), and a mutant (MT), specifically, it is a diagram confirming that a stop codon (asterisk) is formed abnormally early by converting the DNA nucleotide sequence (top) into an amino acid sequence (bottom).

### MODE OF DISCLOSURE

Hereinafter, the present disclosure will be described in more detail through examples. However, these examples are intended to illustrate the present disclosure, and the scope of the present disclosure is not limited to these examples.

### Example

### 1. Production of low-nicotine tobacco by using the CRISPR/Cas9 system

### (1) Selection of nicotine biosynthesis gene and production of gene carrier

QPT1 and QPT2 genes are present in tobacco, and the gene expressed in response to a wound or methyl jasmonate treatment, which are signals for inducing nicotine biosynthesis, is known as QPT2.

Accordingly, in order to produce low-nicotine plant cells, two genes, QPT1 and QPT2, including a quinolinic acid phosphoribosyl transferase (QPT) gene, a gene related to nicotine biosynthesis, were selected.

### (2) Identification of nucleotide sequences of genes related to nicotine biosynthesis (NtQPT1 and NtQPT2)

To identify the gene sequence of NtQPT in a burley tobacco (KB108), which is a research target plant, primers specific for each gene were prepared based on the nucleotide sequence information published in the national center for biotechnology information (NCBI) database, polymerase chain reaction (PCR) was performed, and a sequencing service was requested. The sequences of primers specific for each gene and PCR conditions are shown in Table 1 below.

**[Table 1]**

| Primer information and gene amplification conditions for amplifying a NtQPT gene | | | | | | |
|---|---|---|---|---|---|---|
| Name of Primer | Nucleotide sequence of primer | Am plifi ed size (bp) | Gene amplification condition | | | SEQ ID NO |
| | | | Annealing Tmp (°C) | Elongatio n time (min) | Cycles | |
| F_Q1s | AATAAGCTTGGCCAAATTT TAGGAC | 589 6 | 57 | 35 | 40 | 1 |
| R_Q1s | CAACTTTTTACATTGTGTC AAATTCCATG | | | | | 2 |
| F_Q1t | AGGCATATGTTAATTGTTC GCAAAATC | 631 0 | 62 | 35 | 40 | 3 |
| R_Q1t | CACCAAAACATGAAAGCAA AGCT | | | | | 4 |
| F_Q2s | GTGCTATTGCCTGTTAACT CTCAC | 487 0 | 61 | 25 | 40 | 5 |
| R_Q2s | GAGCAGACACAGAAATTAT TTAGTGACT | | | | | 6 |
| F_Q2t | GGAGTGCTATTGACAGTTA ACTCAC | 432 8 | 63 | 25 | 40 | 7 |
| R_Q2t | AGCTTCAGATACACCAAAC TTGTAAA | | | | | 8 |

As a result of amplifying the gDNA region including exons 1 to 8 of a KB108 species and analyzing the nucleotide sequence, it was confirmed that the nucleotide sequence mostly matched with the published nucleotide sequence. Specifically, the nucleotide sequence of a QPT2 gene confirmed to be derived from *Nicotiana sylvestris* (hereinafter, QPT2s), and a QPT2 gene confirmed to be derived from *Nicotiana tomentosiformis* (hereinafter, QPT2t) could be identified. Specifically, the nucleotide sequence information of NtQPT1 (AJ748262) and NtQPT2 (AJ748263) genes of *N. tabacum,* which is published in the NCBI GenBank batabase is shown in Table 2 below.

**[Table 2]**

| QPT gene sequence information of *N. tabacum* published on NCBI GenBank | | | |
|---|---|---|---|
| NO. | Gene | Gene Accession number | Protein ID |
| 1 | Nicotiana tabacum mRNA for putative quinolinate phosphirobosyltransferase (QPT1 gene) | AJ748262 | CAH04306.1 |
| 2 | Nicotiana tabacum QPT2 gene for putative quinolinate phosphoribosyltransferase, exons 1-10. | AJ748263 | CAH04307.1 |

Accordingly, using the nucleotide sequences of the NtQPT1 and NtQPT2 genes, comparisons were made to find a homologous gene to the NCBI reference sequence, and a list of nucleotide sequences with high homology confirmed through this result is shown in Table 3 below.

**[Table 3]**

| A list of nucleotide sequences with high homology obtained by comparing the nucleotide sequences of two QPT genes published in GenBank with the NCBI Reference Sequence database. | | | | |
|---|---|---|---|---|
| NO | SEQUENCE ID | Gene ID | Species | Anticipated gene |
| 1 | NW_009541109.1 | 10421726 9 | N. sylvestris | QPT1 |
| 2 | NW_009350226 | 10782007 8 | N. sylvestris | QPT2 |
| 3 | NW_008919084.1 | 10412104 6 | N. tomentosiformis | QPT1 |
| 4 | NW_008919267.1 | 10412114 0 | N. tomentosiformis | QPT2 |
| 5 | NW_015842103.1 | 10782584 9 | N. tabacum | QPT1 (QPT1s) derived from *N*. *sylvestris* |
| 6 | NW_015824186.1 | 10424001 4 | N. tabacum | QPT2 (QPT2s) derived from *N*. |
| | | | | *sylvestris* |
| 7 | NW_015852968.1 | 10782912 3 | N. tabacum | QPT1 (QPT1t) derived from *N*. *tomentosiformis* |
| 8 | NW_015852968.1 | 10782912 2 | N. tabacum | QPT2 (QPT2t) derived from *N*. *tomentosiformis* |

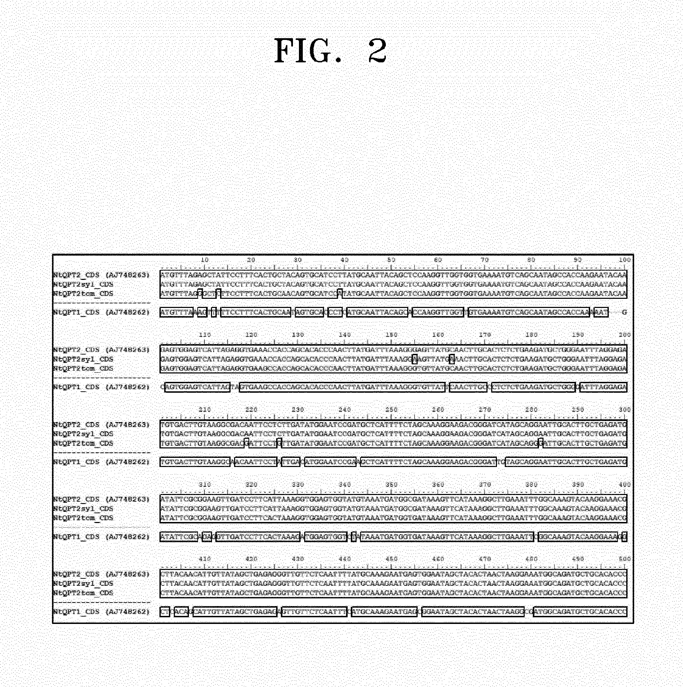

As confirmed in Table 3, 8 sequences with high similarity to the Reference Sequence were identified and selected. Homology between the public QPT gene sequence in Table 2 and nucleotide sequences of CDS regions of the 10 selected QPT gene sequences in Table 3 were compared, and the results are shown in Table 4 and FIG. 2.

**[Table 4]**

| Homology matrix of CDS regions of 10 public QPT genes used in examples | | | | | | | | | | |
|---|---|---|---|---|---|---|---|---|---|---|
| Comparison of homology of QPT genes | AJ748262 | AJ748263 | NW_015842103.1 (107825849) | NW_015852968.1 (107829123) | NW_015824186.1 (104240014) | NW_015852968.1 (107829122) | NW_009541109.1 (104217269) | NW_008919084.1 (104121046) | NW_009350226 (107820078) | NW_008919267.1 (104121140) |
| AJ748262 | ID | 0.94 | **0.99** | 0.97 | 0.94 | 0.94 | **0.99** | 0.97 | 0.94 | 0.94 |
| AJ748263 | 0.94 | ID | 0.94 | 0.93 | **1.00** | 0.98 | 0.94 | 0.93 | **1.00** | 0.98 |
| NW_015842103.1(107825849) | **0.99** | 0.94 | ID | 0.97 | 0.93 | 0.94 | **1.00** | 0.97 | 0.93 | 0.94 |
| NW_015852968.1(107829123) | 0.97 | 0.93 | 0.97 | ID | 0.93 | 0.93 | 0.97 | **1.00** | 0.93 | 0.93 |
| NW_015824186.1(104240014) | 0.94 | **1.00** | 0.93 | 0.93 | ID | 0.98 | 0.93 | 0.93 | **1.00** | 0.98 |
| NW_015852968.1(107829122) | 0.94 | 0.98 | 0.94 | 0.93 | 0.98 | ID | 0.94 | 0.93 | 0.98 | **1.00** |
| NW_009541109.1(104217269) | **0.99** | 0.94 | **1.00** | 0.97 | 0.93 | 0.94 | ID | 0.97 | 0.93 | 0.94 |
| NW_0 | 0.97 | 0.93 | 0.97 | **1.00** | 0.93 | 0.93 | 0.97 | ID | 0.9 | 0. |
| 08919084.1(104121046) | | | | | | | | | 3 | 93 |
| NW_009350226(107820078) | 0.94 | **1.00** | 0.93 | 0.93 | **1.00** | 0.98 | 0.93 | 0.93 | ID | 0.98 |
| NW_008919267.1(104121140) | 0.94 | 0.98 | 0.94 | 0.93 | 0.98 | **1.00** | 0.94 | 0.93 | 0.98 | ID |

As confirmed in Table 4 and FIG. 2 above, NtQPT1 (AJ748263) and NtQPT2 (AJ748262) registered in NCBI GenBank show more than 99 % homology with the QPT1 and QPT2 genes of *N. sylvestris,* respectively, so it was confirmed that they were derived from *N. sylvestris,* and it was found that the nucleotide sequences of QPT1 and QPT2 derived from *N. tomentosiformis* were missing from GenBank. In addition, in the *Nicotiana tabacum* (TN90), QPT1s (gene ID: 107825849), QPT1t (gene ID: 107829123), QPT2s (gene ID: 104240014) and QPT2t (gene ID: 107829122) genes derived from either parental *N. sylvestris* or *N. tomentosiformis* were confirmed to exist.

### (3) Genetic scissors block design and carrier recombination

11 gene-specific sites that are capable of specifically cutting only the QPT target gene were selected, and a gene carrier capable of cleaving three sites in one gene carrier was constructed. Specifically, cleavage positions were selected in the region of exons 1 to 8 of the NtQPT gene. In addition, common sites capable of cutting both the NtQPT2s gene and the NtQPT2t gene were selected and shown in Table 5 below.

**[Table 5]**

| Nucleotide sequence of scissors guides (sgRNA) that specifically bind to the NtQPT2 gene | | | |
|---|---|---|---|
| sgR NA | Nucleotide sequence | SEQ ID NO | QPT gene binding site |
| sg1 | TGTTTAGAGCTATTCCTTTCA | 9 | Exon 1 |
| sg2 | TGTGTAAAATTGCAGGTTGG | 10 | Exon 2 |
| sg3 | AATACAAGAGTGGAGTCATTAG | 11 | Exon 2 |
| sg4 | CCAAGGTCTCTTTAAGAAGAAAA | 12 | Exon 1 |
| sg5 | GCCACCAAGAATACAAGAGTGG | 13 | Exon 2 |
| sg6 | CCAagaatacaagagtggagtc | 14 | Exon 2 |
| sg7 | GCAAAGGAAGACGGGATCATAGCAGG | 15 | Exon 3 |
| sg8 | GCACTTGCTGAGATGATATTCGCGG | 16 | Exon 3 |
| sg9 | aCAACATTGTTATAGCTGAGAGG | 17 | Exon 5 |
| sg10 | ATATCTGCTGCTGGAGGTGTCGG | 18 | Exon 7 |
| sg11 | GATCAATGGGAGGTTTGATACGG | 19 | Exon 8 |

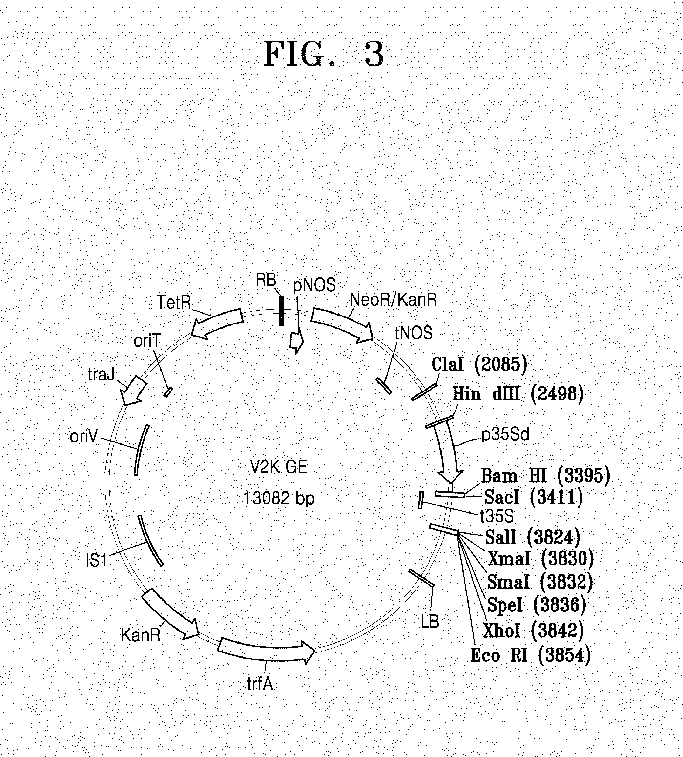

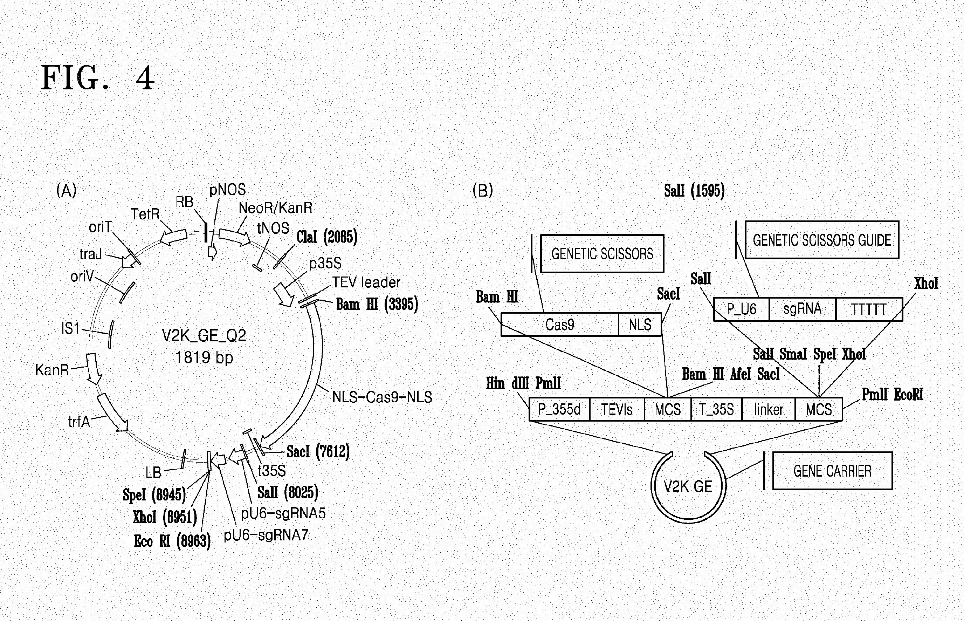

In addition, the prepared plant expression gene carrier (V2k_GE, SEQ ID NO: 20) is shown in FIG. 3, a final genetic carrier (V2k_GE_Q2, SEQ ID NO: 21) prepared by cloning the genetic scissors carrier in the genetic carrier is shown in FIG. 4A, and a scheme of genetic scissors and genetic scissors guide included in the final genetic carrier is shown in FIG. 4B. pBI121 is a binary vector capable of replicating in E. *coli* and agrobacterium and is widely used for plant transformation. Therefore, as specifically confirmed in FIGS. 4A and 4B, pBI121 was cut with Hindlll and EcoRI to prepare to clone the GE_block required for the CRISPR/Cas9 system. A GE_block sequentially consists of a CaMV 35S promoter with dual enhancer (P_35Sd), a TEV leader sequence (TEVIs), a multi cloning site (MCS) for cloning a Cas9 block, a CaMV 35S terminator (T_35S), a linker sequence, and a multi cloning site (MCS) for cloning the sgRNA block, and nucleotide sequences for recognizing Hindlll and EcoRl are added on both ends. Each block of the GE_block was prepared by DNA synthesis and was completed by sequential cloning. V2k_GE was prepared by ligating pBI121 and the GE_block cut with Hindlll and EcoRl.

A Cas9_block is composed of a Cas9 coding sequence (CDS) and C-terminus nuclear localization sequence (NLS), and BamHl and Sacl recognition sequences are added to both ends of the block (Cas9_block); and a block consists of U6 promoter (P_U6), sgRNA, and poly T and as a block which is capable of expressing sgRNA (sgRNA_QPT), and a block consists of a U6 promoter (P_U6), sgRNA, and poly T. sgRNA_QPT was completed by linking 11 types of sgRNA blocks, which are capable of specifically binding to the QPT gene, into one continuous DNA through overlap extension PCR technique. Recognition sequences for Sall and Spel exist at both ends of sgRNA_QPT.

After attaching V2k_GE and Cas9_block cut with BamHl and Sacl through a ligation reaction, V2k_GE_Q2 was prepared by cutting with Sall and Spel and inserting sgRNA_QPT.

### (4) Introduction of recombinant carrier into gene transfer microorganisms (Agrobacterium)

A carrier for plants was transformed into an Agrobacterium LBA4404 strain by the freeze-thaw method.

Specifically, Agrobacterium was inoculated into YEP liquid medium (yeast extract 10 g, bacto peptone 10 g, NacCl 5 g), and then cultured with shaking at 28 °C and 250 rpm for 16 hours. The culture medium was centrifuged at a speed of 3,000 g and 4 °C for 20 minutes to separate the cells, and then suspended in 20 mM of CaCl₂ to prepare competent cells. 100 µL of competent cells were added with 5 µL of plasmid DNA (a carrier for plants), with liquid nitrogen at for 5 minutes, and 37 °C for 5 minutes. 1 mL of YEP liquid medium was added and incubated with shaking under the conditions of 28 °C and 250 rpm for 2 hours. 100 µL of the culture medium was spread on YEP solid medium containing 100 mg/L of kanamycin, and then incubated for 3 days at 28 °C. After subculturing each of single colonies, it was confirmed by PCR whether the plasmid DNA was transformed.

### (5) Plant tissue culture

### 1) Plant transformation

Agrobacterium was cultured in YEP liquid media (including 70 mg/L of kanamycin and 70 mg/L of streptomycin) at 28 °C for 24 hours.

After sterilizing the leaves of a plant one-month-old after the germination with 70 % ethanol and chlorine bleach, the leaves were cut into 3 mm X 3 mm slices, the sections were placed on a Petri dish containing 5 ml of MS liquid medium, 1 mL of Agrobacterium culture solution was sprayed evenly, and the sample was cultured for 48 hours at 25 °C in dark conditions to prepare tobacco leaf sections.

### 2) Plant tissue culture

After washing the leaf sections in sterile distilled water containing 200 µg/ml cefotaxime 4 times, the leaf sections were placed on shooting medium (MS medium including 2 mg/L BA, 0.1 mg/L NAA, 200 mg/L cefotaxime, and 100 mg/L kanamycin) and cultured under the conditions of 25 °C and 16 hours / 8 hours photoperiods, subcultured with fresh medium every 2 weeks, and then placed on washing and selection medium.

In addition, differentiated shoots were cut from the leaf sections and placed in rooting medium (MS medium containing 200 mg/L cefotaxime), and cultured under the conditions of 25 °C and 16 hours / 8 hours photoperiods.

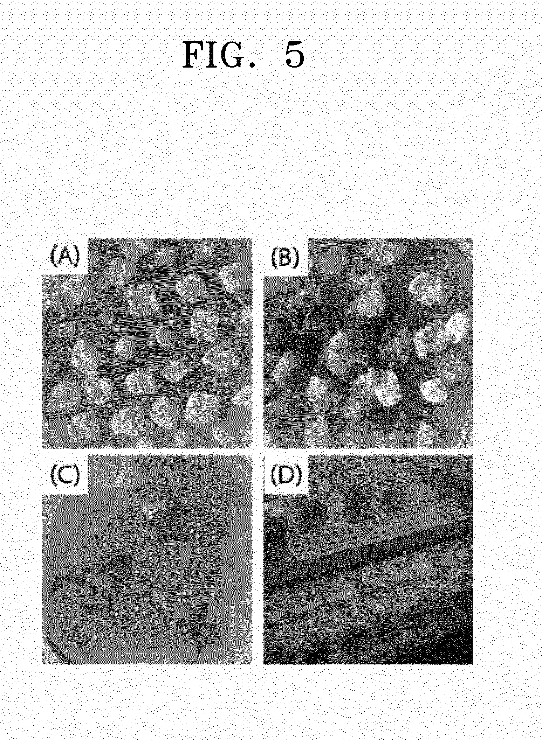

After transforming a tobacco leaf tissue by the Agrobacterium mediated transformation method as described above (FIG. 5A), it was confirmed that callus differentiation (FIG. 5B), leaf differentiation (FIG. 5C), and root differentiation (FIG. 5D) were performed well in sequence, and 57 tissue-cultured plantlets with roots were obtained.

### (6) Selection of F0 to F1 generation mutants

### 1) Confirmation of mutation in the target gene and its pattern

100 mg of healthy leaf tissue was sampled and uniformly pulverized, and then genomic DNA was extracted and purified by using a commercial kit (for example, Nucleospin 96 plant II, Macherey Nagel, Germany) using a silica column. After gDNA was extracted/purified from the leaf tissue, the target gene site was amplified through PCR and the nucleotide sequence was analyzed.

Specifically, by amplifying each of QPT2s and QPT2t genes of tissue cultures and analyzing the nucleotide sequence, occurrence and pattern of mutations through guide sequences of sg 1 to sg11 were confirmed, and the results are shown in Table 6.

**[Table 6]**

| sgRNA | Number of mutations per 50 tissue cultures |
|---|---|
| sg1 | 0 |
| sg2 | 1 |
| sg3 | 0 |
| sg4 | 1 |
| sg5 | 18 |
| sg6 | 2 |
| sg7 | 3 |
| sg8 | 2 |
| sg9 | 1 |
| sg10 | 2 |
| sg11 | 3 |

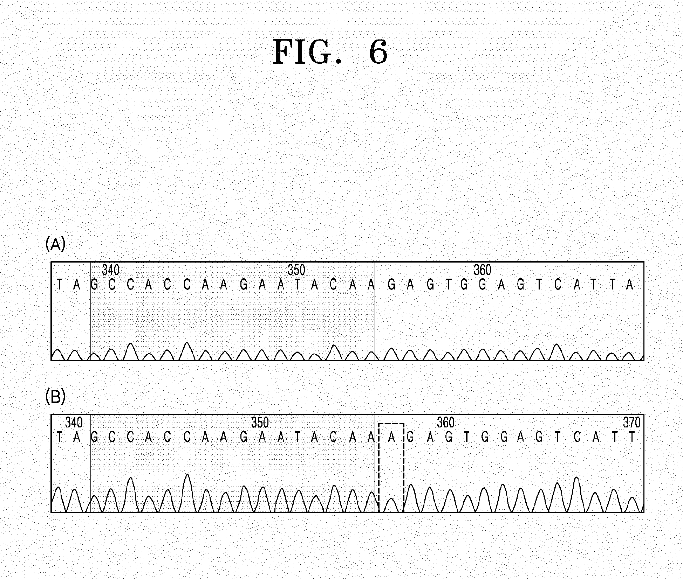

As confirmed in Table 6, it was confirmed that mutations were frequently induced in the nucleotide sequence of the sg5 sgRNA binding site. In addition, it was confirmed that mutations were induced in the order of sg7 and sg11. Afterwards, selection of 8 types of tissue cultures in which mutations in the QPT2 gene were induced were completed, mutation patterns in the QPT (QPT2s and QPT2t) genes of the eight tissue cultures are shown in Table 7 below, and QPT2s gene sequencing profiles of the wild type (FIG. 6A) and the mutant No. 9 in Table 7 (FIG. 6B) are shown in FIGS. 6A and 6B.

**[Table 7]**

| NO | Subject No. | QPT2s | | QPT2t | |
|---|---|---|---|---|---|
| | | sg5 position | sg7 position | sg5 position | sg7 position |
| 1 | 1 | Hetero | Wild | Hetero | Wild |
| 2 | 4 | Hetero | Wild | Wild | Wild |
| 3 | 5 | Hetero | Wild | Wild | Wild |
| 4 | 7 | Hetero | Wild | Wild | Wild |
| 5 | 9 | Dual | Wild | Hetero | Wild |
| 6 | 10 | Dual | Wild | Wild | Wild |
| 7 | 12 | Hetero | Wild | Wild | Wild |
| 8 | 20 | Wild | Wild | Homo | Wild |

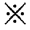 Dual: indicates that different types of mutations are generated in two alleles.
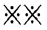 Hetero: indicates that one allele of the two alleles is mutated and the other allele is not mutated.
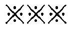 Homo: indicates that the same type of mutation is generated in both alleles
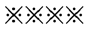 Wild: indicates that mutation did not occur.

### 2) Acclimatization of plants

Eight tissue cultures with confirmed mutations were transplanted into pots containing top soil and grown in a greenhouse.

### 3) Securing F1 generation seeds and confirming removal of transgenes

F1 generation seeds were obtained through self-fertilization to remove the gene block introduced for CRISPR/Cas9 expression. After sowing seeds of plant number 9 among the tissue cultures and culturing for 30 days in a 192-cell tray, the leaves were collected and ground evenly, and then genomic DNA was extracted and purified by using a commercial kit (for example, Nucleospin 96 plant II, Macherey Nagel, Germany) using a silica column. Presence of a Cauliflower mosaic virus (CaMV) 35S promoter was checked using the real-time PCR technique of the TaqMan probe method, and plants from which external transgenes were removed were selected.

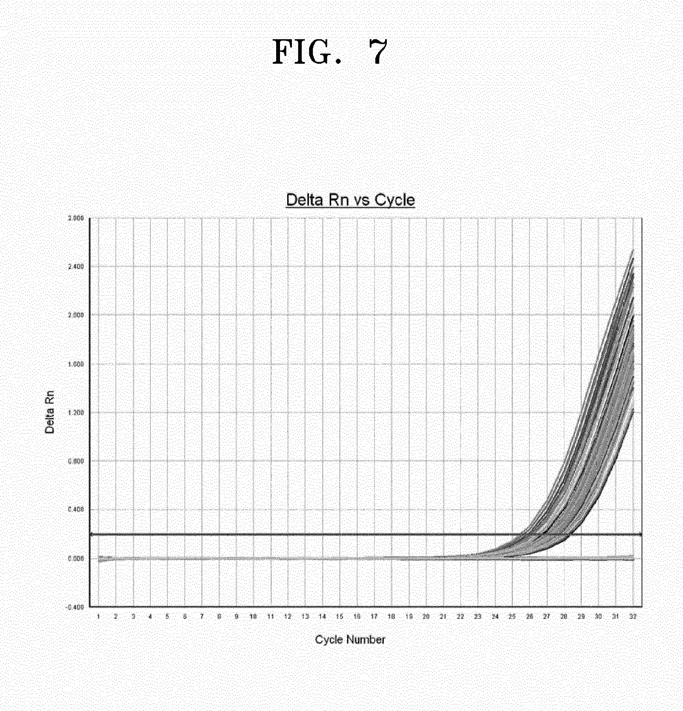

Presence or absence of a p35S promoter in 192 F₁ generation plants grown by sowing seeds of plant number 9 among the tissue cultures was confirmed by real-time PCR, and the results are shown in FIG. 7. As confirmed in FIG. 7 , it was confirmed that there was no p35S promoter in 48 plants.

### 4) Selection of QPT homozygous mutants from finally selected F₁ generation plants

Through Example (6)-3), among the p35S- plants showing mutations, a plant MT_QPT2st_F1, in which homozygous mutations were induced in both of the target genes of QPT2s and QPT2t genes, was finally selected. Thereafter, mutation patterns for the QPT2s and QPT2t genes of the finally selected plant were analyzed, and the results are shown in Table 8.

The primers used to confirm the mutation pattern of the QPT2 gene in the analysis method are shown in Table 1 above. Specifically, the F_Q2s and R_Q2s primers in Table 1 were used to specifically amplify the QPT2s gene, and the F_Q2t and R_Q2t primers in Table 1 were used to amplify the QPT2t gene. In addition, R1q_Q2 primers were commonly used for sequencing, and in this case, the nucleotide sequence of R1q_Q2 used was GGCCACATATTAGGTAATTACA (SEQ ID NO: 32).

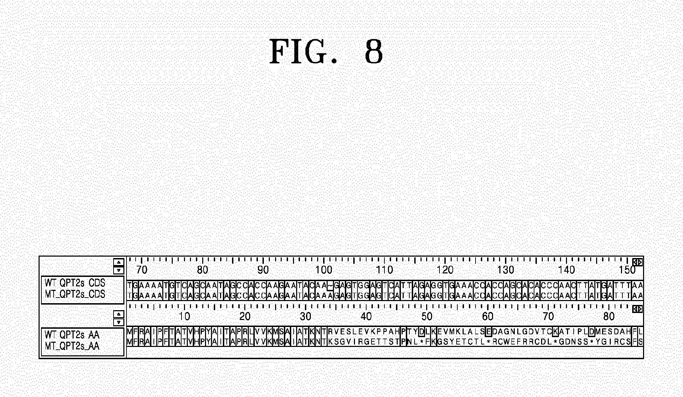

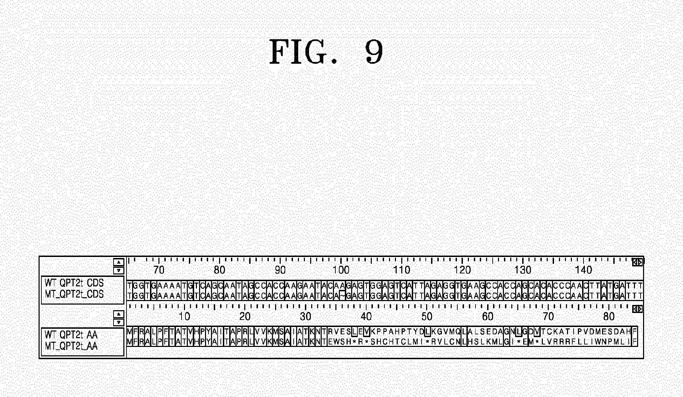

Mutation patterns of a QPT2s gene of a burley species control group (WT), and MT_QPT2st_F1 mutant (MT) were shown in FIG. 8, and mutation patterns of a QPT2t gene of a burley species control group (WT), and MT_QPT2st_F1 mutant (MT) were shown in FIG. 9.

**[Table 8]**

| Variety | **Mutation in QPT2s** gene | **Mutation in QPT2t** gene |
|---|---|---|
| KB108(MT_QPT2st_F1) | **Homo mutation(A** ins) | **Homo** mutation **(A del)** |

As confirmed in FIGS. 8 and 9, as a result of aligning DNA sequences of QPT2s and QPT2t genes of MT_QPT2st_F1 with the nucleotide sequence of a wild-type burley tobacco, and converting the DNS sequence into an amino acid sequence and comparing the same with the amino acid sequences of QPT2s and QPT2t, it was confirmed that a stop codon was generated abnormally early in the mutant (MT_QPT2st_F1).

### (7) Analysis of the final mutant genotype

The F₁ plant (MT_QPT2st_F1) selected in the above example was crossed with the wild-type KB108 variety to obtain an F₂ hybrid variety. Afterwards, from among the F₃ plants obtained by self-crossing, each of plants in which mutation is induced only in the QPT2s gene, plants in which mutation is induced only in the QPT2t gene, and plants in which mutation is induced both in the QPT2s gene and the QPT2t gene were selected, and mutation patterns of the finally selected mutants of the F₁ to F₃ generations are summarized in Table 9 below.

**[Table 9]**

| Variety | Generation | Mutation in QPT2s gene | Mutation in QPT2t gene |
|---|---|---|---|
| MT_QPT2st_F1 | F1 generation | Homo mutation(A ins) | Homo mutation (A del) |
| MT_QPT2st_F1 X KB108 (Wild-type) | F2 generation | Hetero mutation(A ins / wild) | Hetero mutation (A del / wild) |
| MT_QPT2s | F3 generation | Homo mutation(A ins) | Wild type |
| MT_QPT2t | F3 generation | Wild type | Homo mutation(A del) |
| MT_QPT2st | F3 generation | Homo mutation(A ins) | Homo mutation (A del) |

### 2. Analysis of nicotine content of finally selected F₃ generation QPT mutants

An experiment was performed to identify specifically which gene among the QPT2 genes contributes to reducing the contents of nicotine and alkaloids in tobaccos when inactivated. In particular, among the F₃ plants prepared in the above examples, there are plants in which mutation is induced only in the QPT2s gene, plants in which mutation is induced only in the QPT2t gene, and plants in which mutation is induced both in the QPT2s gene and the QPT2t gene, thus, contents of nicotine and alkaloids in the finally selected MT_QPT2s, MT_QPT2t and MT_QPT2st were identified.

After cutting the flower stalks of plants grown for 60 days in a greenhouse environment, all leaves of F0 generation plants were harvested two weeks later. After the harvested leaves were dried in a dry oven at 65 °C for 48 hours, the dried leaves were placed in a container with glass beads and pulverized by using a gyro-shaker. Thereafter, analyses of nicotine content and alkaloid content were performed through GC/MS analysis, and the results are shown in Table 10.

**[Table 10]**

| Variety | Nicotine 1) (mg/g) | Nornicotin e (mg/g) | Anabasi ne (mg/g) | Anatabin e (mg/g) |
|---|---|---|---|---|
| WT | 24.55 | 0.51 | 0.14 | 0.72 |
| MT_QPT 2s | 25.98 | 0.46 | 0.12 | 0.51 |
| MT_QPT 2t | 20.12 | 0.46 | 0.11 | 0.50 |
| MT_QPT 2st | 0.6 | 0.16 | N.D. ²⁾ | N.D. |

### 1) Nicotine content (mg) per dry weight (g) of leaves, 2) ND (Not Detected): less than the detection limit value (0.0002 mg/g)

As confirmed in Table 10, as a result of quantitative analysis of the nicotine content of KB108 (wild type: WT) and the three mutants, nicotine content of the wild-type (WT) was 24.55 mg/g on average based on the weight of dry leaves, the mutants of QPT2s alone did not show a decrease in nicotine content, and the mutants of QPT2t alone showed nicotine content of 20.12 mg/g on average, which was slightly reduced than that of the wild type. On the other hand, in MT_QPT2st with mutations in QPT2s and QPT2t, it was confirmed that the nicotine content was significantly reduced to an average of 0.6 mg/g.

In addition, with respect to other alkaloids other than nicotine, it was found specifically that in MT_QPT2st with mutations in QPT2s and QPT2t compared to the wild-type, alkaloids other than nicotine, anabasine and anatabine were below the detection limit, and the content of nornicotine was also significantly reduced.

## Claims

1. A plant cell genetically engineered to have reduced expression or activity of a quinolinic acid phosphoribosyl transferase (QPT) gene or a QPT protein, compared to the parent cell.

2. The plant cell of claim 1, wherein the QPT gene is at least one gene selected from the group consisting of QPT2s and QPT2t.

3. The plant cell of claim 1, wherein the plant cell is genetically engineered by at least one selected from the group consisting of an RNA interference (RNAi) system, a meganuclease system, a zinc finger nuclease system, and a TALEN system, a CRISPR/Cas system, X-ray irradiation, gamma-ray irradiation, ethyl methanesulfonate treatment, and dimethyl sulfate treatment.

4. The plant cell of claim 3, wherein the CRISPR/Cas system comprises: at least one first polynucleotide selected from the group consisting of polynucleotides including nucleotide sequences of SEQ ID NOS: 9 to 19; or a second polynucleotide into which the at least one first polynucleotide is transcribed.

5. The plant cell of claim 4, wherein the second polynucleotide is sgRNA comprising CRISPR RNA (crRNA) and transactivating crRNA (tracrRNA).

6. The plant cell of claim 4, wherein the second polynucleotide is bound to at least one site in the region consisting of Exons 1 to 8 of the QPT gene.

7. The plant cell of claim 4, wherein the CRISPR/Cas system comprises: a Cas protein selected from the group consisting of Cas1, Cas1B, Cas2, Cas3, Cas4, Cas5, Cas6, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and Cpf1, or a gene encoding the Cas protein; and a nuclear localization signal (NLS) protein or a gene encoding an NLS protein.

8. The plant cell of claim 1, wherein the plant is *Nicotiana tabacum.*

9. A plant comprising any one plant cell of claims 1 to 8.

10. A CRISPR/Cas system comprising: at least one first polynucleotide selected from the group consisting of polynucleotides including nucleotide sequences of SEQ ID NOS: 9 to 19; or a second polynucleotide into which the at least one first polynucleotide is transcribed.

11. The CRISPR/Cas system of claim 10, wherein the system is one that genetically engineers to reduce expression or activity of a QPT gene or a QPT protein in plant cells, and the QPT gene is a QPT gene (NtQPTs) derived from *Nicotiana sylvestris,* and a QPT gene (NtQPTt) derived from *Nicotiana tomentosiformis* or a combination of NtQPTs and NtQPTt.

12. A composition for inhibiting alkaloid biosynthesis comprising the CRISPR/Cas system of claim 10 or 11, wherein the alkaloid is at least one selected from the group consisting of nornicotine, anatabine and anabasine.

13. A composition for inhibiting nicotine biosynthesis comprising the CRISPR/Cas system of claim 10 or 11.

14. A composition for genetically engineering QPT including the CRISPR/Cas system of claim 10 or 11.

15. A method of producing plant cells in which nicotine biosynthesis is inhibited comprising introducing into a plant cell a vector including a CRISPR/Cas system including: at least one first polynucleotide selected from the group consisting of polynucleotides including nucleotide sequences of SEQ ID NOS: 9 to 19; or a second polynucleotide into which the at least one first polynucleotide is transcribed.

16. A method of genetically engineering QPT of plant cells comprising introducing into a plant cell a vector comprising a CRISPR/Cas system including: at least one first polynucleotide selected from the group consisting of polynucleotides including nucleotide sequences of SEQ ID NOS: 9 to 19; or a second polynucleotide into which the at least one first polynucleotide is transcribed.
